(19) Europäisches Patentamt
European Patent Office
Office européen des brevets

(11) Publication number: 0 227 284 A1

(12) **EUROPEAN PATENT APPLICATION**

(21) Application number: 86308759.9

(22) Date of filing: 11.11.86

(51) Int.Cl.[4]: C 07 D 249/12
A 01 N 43/653
//C07C133/02, C07C159/00

(30) Priority: 12.11.85 US 797304

(43) Date of publication of application:
01.07.87 Bulletin 87/27

(84) Designated Contracting States:
AT BE CH DE ES FR GB GR IT LI LU NL SE

(71) Applicant: ELI LILLY AND COMPANY
Lilly Corporate Center
Indianapolis Indiana 46285(US)

(72) Inventor: Beck, James Richard
10023 Penrith Drive
Indianapolis Indiana 46229(US)

(74) Representative: Tapping, Kenneth George et al,
Erl Wood Manor
Windlesham Surrey, GU20 6PH(GB)

(54) **Plant Growth regulating triazoles.**

(57) Triazole compounds of the formula I

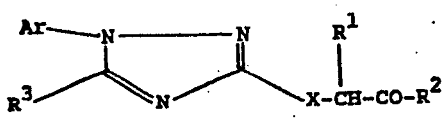

(I)

wherein

X is –O– or –S–;

Ar is phenyl or phenyl substituted with 1 or 2 groups independently selected from halo, trifluoromethyl, $C_1$-$C_3$ alkyl, $C_1$-$C_3$ alkoxy, difluromethoxy, trifluoromethoxy, pentafluoroethoxy, and 1,1,2,2-tetrafluoroethoxy;

$R^1$ is $C_1$-$C_4$ primary or secondary alkyl;

$R^2$ is hydroxy, $C_1$-$C_4$ alkoxy, benzyloxy, phenoxy, or $-NR^4R^5$;

$R^3$ is hydrogen or $C_1$-$C_4$ primary or secondary alkyl;

$R^4$ and $R^5$ independently are hydrogen, $C_3$-$C_6$ cycloalkyl or $C_1$-$C_3$ alkyl, or $R^4$ and $R^5$ combine with the nitrogen atom to which they are attached to form morpholino, pyrrolidino or piperidino; and phytologically-acceptable salts thereof are plant growth regulators.

EP 0 227 284 A1

# PLANT GROWTH REGULATING TRIAZOLES

Workers in the field of agricultural chemistry and plant physiology have known for some time that certain chemicals are able to control plant behavior in beneficial ways. For example, compounds have been found which modify plant growth, enhance the yield of crops, improve the quality of crops, or improve mechanical harvesting operations by altering the growth habit of plants. Compounds which are applied to a plant to alter its processes or structure in a beneficial way are called plant growth regulators. Some instances of goals of plant growth regulation are the slowing of growth of turf to decrease the frequency of mowing, the shortening of grain stems to decrease lodging, causing ornamental plants to be more compact, increasing flowering of fruit-bearing crops, increasing fruit set of tomatoes, and reducing the size of crop plants so that they can be planted more densely.

The present invention provides triazole compounds of the formula

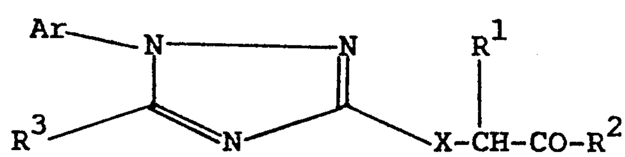

wherein

X is -O- or -S-;

Ar is phenyl or phenyl substituted with 1 or 2 groups independently selected from halo, trifluoromethyl, $C_1$-$C_3$ alkyl, $C_1$-$C_3$ alkoxy, difluromethoxy, trifluoromethoxy, pentafluoroethoxy, and 1,1,2,2-tetrafluoroethoxy;

$R^1$ is $C_1$-$C_4$ primary or secondary alkyl;

$R^2$ is hydroxy, $C_1$-$C_4$ alkoxy, benzyloxy, phenoxy, or $-NR^4R^5$;

$R^3$ is hydrogen or $C_1$-$C_4$ primary or secondary alkyl;

$R^4$ and $R^5$ independently are hydrogen, $C_3$-$C_6$ cycloalkyl or $C_1$-$C_3$ alkyl, or $R^4$ and $R^5$ combine with the nitrogen atom to which they are attached to form morpholino, pyrrolidino or piperidino; or a phytologically-acceptable salt thereof.

The invention also provides agricultural compositions comprising the above compounds and a phytologically-acceptable diluent, and further provides a method of regulating the growth of plants which comprises applying an effective amount of a compound of the above formula to the plant to be regulated at a time not later than the late reproductive growth stage.

Throughout the present document, all temperatures will be expressed in degrees Celsius. All expressions of concentration, proportion and the like will refer to weight units unless otherwise stated.

In the above formula, the general chemical terms have their usual meanings. For example, the terms $C_1$-$C_3$ alkyl and $C_1$-$C_3$ alkoxy include the groups methyl, ethyl, propyl, and isopropyl, which may be linked through an oxygen atom to form the alkoxy groups. The term $C_1$-$C_4$ alkoxy includes the above alkoxy groups, and also includes butoxy, 1-methylpropoxy, 2-methylpropoxy

and t-butoxy. The term $C_1$-$C_4$ primary or secondary alkyl includes methyl, ethyl, propyl, isopropyl, butyl, 1-methylpropyl and 2-methylpropyl. The term $C_3$-$C_6$ cycloalkyl includes such groups as cyclopropyl, cyclobutyl and cyclohexyl.

The term halo includes fluoro, chloro, bromo and iodo.

The above formula describes the compounds of the present invention, but the following group of compounds are specifically mentioned to assure that the invention is easily understood.

3-(1-carboxyethoxy)-1-(2-fluorophenyl)-1,2,4-1H-triazole

3-(1-carboxypropoxy)-1-(4-chlorophenyl)-1,2,4-1H-triazole, sodium salt

1-(3-bromophenyl)-3-(1-methoxycarbonylbutylthio)-5-methyl-1,2,4-1H-triazole

3-(1-ethoxycarbonyl-2-methylpropoxy)-1-(4-iodophenyl)-1,2,4-1H-triazole

5-ethyl-3-(1-propoxycarbonylpentylthio)-1-(3-trifluoromethylphenyl)-1,2,4-1H-triazole

3-(1-isopropoxycarbonyl-2-methylbutoxy)-5-methyl-1-(3-methylphenyl)-1,2,4-1H-triazole

3-(1-butoxycarbonyl-3-methylbutoxy)-1-(2-ethylphenyl)-5-methyl-1,2,4-1H-triazole

3-(1-isobutoxycarbonylethoxy)-5-isopropyl-1-(4-isopropylphenyl)-1,2,4-1H-triazole

3-(1-s-butoxycarbonylethoxy)-1-(4-methoxyphenyl)-5-methyl-1,2,4-1H-triazole

3-(1-t-butoxycarbonylpropoxy)-1-(3-ethoxyphenyl)-5-propyl-1,2,4-1H-triazole

3-(1-benzyloxycarbonylethoxy)-1-(2-isopropoxyphenyl)-5-methyl-1,2,4-1H-triazole

5-butyl-3-(1-carboxyethylthio)-1-(3,5-difluorophenyl)-1,2,4-1H-triazole, potassium salt

1-(2,4-dibromophenyl)-5-methyl-3-(1-phenoxycarbonylbutoxy)-1,2,4-1H-triazole

3-(1-aminocarbonylethoxy)-1-[3,5-bis(trifluoromethyl)phenyl]-1,2,4-1H-triazole

3-(1-methylaminocarbonylethoxy)-1-(2,5-dimethylphenyl)-1,2,4-1H-triazole

3-(1-ethylaminocarbonylethylthio)-1-(2,6-diethylphenyl)-1,2,4-1H-triazole

5-(1-methylpropyl)-1-[3,4-bis(methoxy)phenyl]-3-(1-propylaminocarbonylpropoxy)-1,2,4-1H-triazole

3-(1-isopropylaminocarbonylethoxy)-1-[2,4-bis(propoxy)phenyl]-1,2,4-1H-triazole

3-(1-ethylmethylaminocarbonyl-2-methylpropoxy)-1-(2-fluoro-4-trifluoromethylphenyl)-1,2,4-1H-triazole

1-(3-bromo-5-chlorophenyl)-5-isobutyl-3-(1-dipropylaminocarbonylethoxy)-1,2,4-1H-triazole

3-(1-ethylpropylaminocarbonylethylthio)-1-(4-fluoro-3-iodophenyl)-1,2,4-1H-triazole

1-(3-chloro-4-ethylphenyl)-3-(1-isopropylmethylaminocarbonylpentoxy)-1,2,4-1H-triazole

1-(2-bromo-4-isopropylphenyl)-3-(1-morpholinocarbonylethoxy)-1,2,4-1H-triazole

1-(2-fluoro-5-methylphenyl)-3-(1-pyrrolidinocarbonylethoxy)-1,2,4-1H-triazole

1-(2-chloro-3-methoxyphenyl)-5-methyl-3-(1-piperidinocarbonyl-2-methylbutylthio)-1,2,4-1H-triazole

3-(1-carboxyethoxy)-1-(2-fluoro-4-isopropoxyphenyl)-1,2,4-1H-triazole

3-(1-methoxycarbonylethoxy)-1-(4-methyl-3-trifluoromethylphenyl)-1,2,4-1H-triazole

3-(1-carboxyethoxy)-1-(2-propyl-4-trifluoromethylphenyl)-1,2,4-1H-triazole, triethylamine salt

1-(3-ethoxy-5-trifluoromethylphenyl)-3-(1-propoxycarbonyl-3-methylbutylthio)-1,2,4-1H-triazole

3-(1-aminocarbonylethoxy)-1-(4-isopropoxy-2-trifluoromethylphenyl)-1,2,4-1H-triazole

1-(4-methoxy-3-methylphenyl)-5-methyl-3-(1-phenoxycarbonylethylthio)-1,2,4-1H-triazole

3-(1-carboxypropoxy)-1-(5-ethoxy-2-propylphenyl)-1,2,4-1H-triazole, pyridine salt

3-(1-carboxyethoxy)-1-(4-isopropoxy-2-isopropylphenyl)-1,2,4-1H-triazole

1-(4-chlorophenyl)-3-(1-cyclopropylaminocarbonylpropylthio)-1,2,4-1H-triazole

3-[1-(cyclobutyl)(methyl)aminocarbonylethylthio] 1-(3-trifluoromethylphenyl)-1,2,4-1H-triazole

3-(1-cyclohexylaminocarbonylethoxy)-1-(3,5-dichlorophenyl)-1,2,4-1H-triazole

3-[1-(cyclopentyl)(ethyl)aminocarbonyl-2-methylpropoxy]-5-methyl-1-(3,5-dimethylphenyl)-1,2,4-1H-triazole

3-[1-bis(cyclobutyl)aminocarbonylethylthio]-1-phenyl-1,2,4-1H-triazole

1-(4-chloro-3-methylphenyl)-3-(1-cyclopropylaminocarbonylpropoxy)-5-ethyl-1,2,4-1H-triazole

3-(1-cyclohexylaminocarbonylbutylthio)-1-(3-methoxyphenyl)-1,2,4-1H-triazole

3-(1-carboxyethoxy)-1-(3,5-dichlorophenyl)-1,2,4-1H-triazole, triethanolamine salt

3-(1-carboxyethoxy)-1-(3,5-dimethylphenyl)-1,2,4-1H-triazole

While all of the compounds of the present invention are useful, certain sub-classes of the compounds are particularly preferred.

One such preferred class includes compounds of the formula

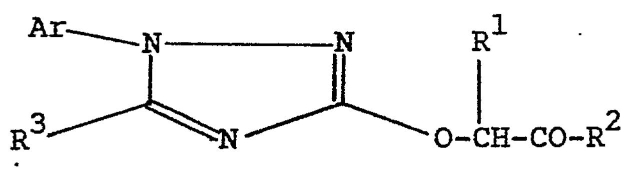

wherein

Ar is phenyl or phenyl substitued with 1 or 2 groups independently selected from halo, trifluoromethyl, $C_1$-$C_3$ alkyl or $C_1$-$C_3$ alkoxy;

$R^1$ is $C_1$-$C_4$ primary or secondary alkyl;

$R^2$ is hydroxy, $C_1$-$C_4$ alkoxy, benzyloxy, phenoxy, or $-N(R^6)(R^7)$;

$R^3$ is hydrogen or $C_1$-$C_4$ primary or secondary alkyl;

$R^6$ and $R^7$ independently are hydrogen or $C_1$-$C_3$ alkyl, or $R^6$ and $R^7$ combine with the nitrogen atom to which they are attached to form morpholino, pyrrolidino or piperidino; and phytologically acceptable salts thereof.

A particularly preferred class includes those compounds wherein X is O, $R^1$ is methyl, $R^2$ is OH or ($C_1$-$C_4$) alkoxy, $R^3$ is hydrogen, and Ar is 3-chlorophenyl, 4-chlorophenyl, or 3,4-dichlorophenyl.

Synthesis

The compounds of the present invention are readily and economically prepared by processes which are analogous to well known synthetic steps. The processes will be briefly discussed in general here, and the preparative examples which follow illustrate in detail the synthesis of the compounds and their intermediates.

The first intermediate for the compounds is a 1-phenylsemicarbazide, having the substituents on the phenyl ring which will characterize the desired product. The semicarbazide is readily obtained by reacting the corresponding phenylhydrazine, preferably in the form of a hydrohalide salt, with a cyanate, preferably an alkali metal cyanate. The process can be readily carried out in water at a moderately elevated temperature in the range of 50-100°, when the preferred forms of the reactants are used. Moderate reaction times such as a few hours are adequate to produce excellent yields.

When a compound wherein X is -S- is to be made, the intermediate is, of course, a 1-phenylthiosemicarbazide, prepared from a thiocyanate.

The above step, as well as the other steps to be described below, may be operated either to maximize yield or to maximize throughput of the process, as may be desirable in the circumstances. The most preferred reaction conditions will differ, of course, depending on which goal is desired. However, it will be found that the synthetic steps are readily carried out to maximize either yield or throughput, and that it is not necessary to use large excess amounts of any reactant in order to obtain good utilization of the other reactants.

The second intermediate in the present synthesis is a 3-hydroxy(thiono)-1-aryl-1,2,4-1H-triazole, which is prepared by reacting the (thio)semicarbazide with an orthoester. When the desired product has no 5-substituent ($R^3$ is hydrogen) the orthoester is an orthoformate. Formic acid may be used instead of an orthoformate. If the product is to have a 5-substituent, the orthoester is chosen to provide the desired substituent. For example, use of an orthoacetate gives a 5-methyl product; use of an orthobutyrate gives a 5-propyl product.

Excellent results in the preparation of the hydroxy(thiono)triazole have been obtained when the orthoester was used in sufficient amount to serve as the reaction solvent, and a small amount of p-toluenesulfonic acid was used as a reaction initiator. Elevated temperatures in the range of 80-120° are suitable. However, the step may also be carried out in an inert solvent, such as, for example, an amide or sulfoxide solvent, using only an approximately stoichiometric amount of the orthoester. Of course, the process may be operated at a temperature above the boiling point of the solvent by carrying out the step under pressure.

The hydroxy(thiono)triazoles are converted to esters of the present invention (wherein $R^2$ is alkoxy) by reacting them with an intermediate of the formula

$$X-\overset{\overset{\displaystyle R^1}{|}}{C}H-CO_2Alk$$

wherein X is chloro or bromo, preferably bromo, and Alk is $C_1$-$C_4$ alkyl, most conveniently ethyl. The reaction is carried out in an inert solvent, most preferably in an amide or sulfoxide solvent, in the

presence of a strong base. The preferred solvents are dimethylsulfoxide, dimethylformamide and dimethylacetamide, and the preferred strong bases are the alkali metal hydrides and alkoxides. The reaction is economically carried out at elevated temperatures in the range of about 80-150°, under pressure if it is economically practical to do so.

When other products of the present invention are desired, the esters prepared in the step which has just been described are easily converted. It is possible to convert such an ester directly to an amide by reacting it with the appropriate substituted amine or with ammonium hydroxide. Such reactions are comparatively slow but may be used if desired. The reactions can be carried out in any convenient solvent, such as an amide, ketone, sulfoxide or the like, at ambient temperature or at a moderately elevated temperature up to about 100°.

The esters are readily hydrolyzed to the corresponding carboxylic acids. The most convenient hydrolyzing agents are strong inorganic bases such as the alkali metal hydroxides and carbonates, especially the hydroxides. Aqueous alkanols such as methanol, ethanol and the like are conventionally used for hydrolysis and are preferred. Operation at the reflux temperature of the reaction medium gives relatively quick hydrolysis to prepare the acid in high and economical yields.

The carboxylic acid may easily be converted to any desired salt, ester or amide of the present invention. The acids are esterified in the usual ways, for example, by reaction with the appropriate alcohol.

Often, it is convenient to first convert the acid to an acid halide, as by reaction with thionyl chloride, thionyl bromide, phosphorus oxychloride or the like, and then to react with the alcohol. The esterification may be carried out directly, by reacting with the alcohol in the presence of an inorganic acid, especially sulfuric acid, or in the presence of a coupling agent such as carbonyldiimidazole. The acid halide may be isolated, or may be formed and further reacted in the same reaction mixture. Inexpensive inert solvents such as aromatics and the like are adequate reaction solvents for the process. Alternatively, the acids may be esterified with a halide of the desired $R^2$ group, carrying out the reaction in the presence of a strong base such as an alkali metal alkoxide.

Finally, the carboxylic acids are converted to the corresponding amides by reaction with the appropriate substituted amine or with ammonium hydroxide. It is preferred to use a coupling agent, such as carbonyldiimidazole, in such a reaction step, and to carry the process out in a highly inert solvent such as an amide, of which such solvents as dimethylformamide and dimethylacetamide are preferred. The reactions go conveniently at ambient temperature in moderate periods of time.

Salts of the carboxylic acids are easily prepared in the usual manner, by simply contacting the compound with the appropriate base in an aqueous alkanol or aqueous ketone. For example, alkali metal salts are obtained by using a base such as sodium, lithium or potassium hydroxide, carbonate or bicarbonate. Amine salts are obtained by using an amine base such as

triethylamine, triethanolamine, pyridine, butyldimethylamine and the like. Salts are prepared at moderate temperatures in the range of 0-100°.

Accordingly, the invention also provides a process for preparing a compound of the formula (I)

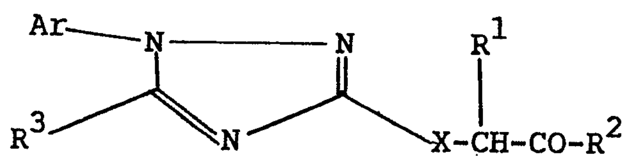 (I)

wherein

X is -O- or -S-;

Ar is phenyl or phenyl substituted with 1 or 2 groups independently selected from halo, trifluoromethyl, $C_1$-$C_3$ alkyl, $C_1$-$C_3$ alkoxy, difluromethoxy, trifluoromethoxy, pentafluoroethoxy, and 1,1,2,2-tetrafluoroethoxy;

$R^1$ is $C_1$-$C_4$ primary or secondary alkyl;

$R^2$ is hydroxy, $C_1$-$C_4$ alkoxy, benzyloxy, phenoxy, or $-NR^4R^5$;

$R^3$ is hydrogen or $C_1$-$C_4$ primary or secondary alkyl;

$R^4$ and $R^5$ independently are hydrogen, $C_3$-$C_6$ cycloalkyl or $C_1$-$C_3$ alkyl, or $R^4$ and $R^5$ combine with the nitrogen atom to which they are attached to form morpholino, pyrrolidino or piperidino; or a phytologically-acceptable salt thereof, which comprises:

a) reacting a compound of formula (II)

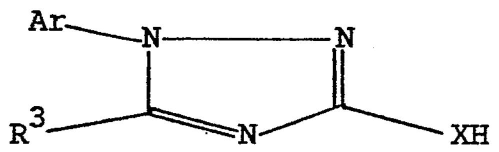 (II)

wherein Ar, $R^3$, and X are as defined above, with a compound of the formula (III)

$$Y-\overset{R^1}{CH}-CO_2-Alk \quad (III)$$

wherein $R^1$ is as defined above, Y is chloro or bromo, and Alk is $C_1$-$C_4$ alkyl, in the presence of a strong base, to produce a compound of formula (I) wherein $R^2$ is $C_1$-$C_4$ alkoxy: or

b) hydrolyzing a compound of formula (I) wherein $R^2$ is $C_1$-$C_4$ alkoxy to provide a compound of formula (I) wherein $R^2$ is OH; or

c) reacting a compound of formula (IV)

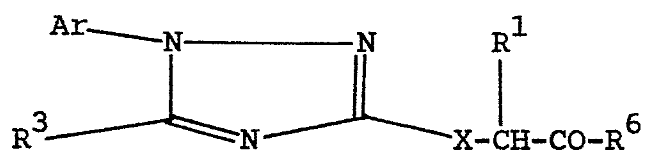 (IV)

wherein Ar, $R^1$, $R^3$, and X are as defined above and $R^6$ is OH or Cl with a $C_1$-$C_4$ alcohol to provide a compound of formula (I) wherein $R^2$ is $C_1$-$C_4$ alkoxy; or

d) reacting a compound of formula (I) wherein $R^2$ is OH or $C_1$-$C_4$ alkoxy with ammonium hydroxide or an amine of the formula $NHR^4R^5$ where $R^4$ and $R^5$ are as defined above, to produce a compound of formula (I) wherein $R^2$ is $NR^4R^5$; and

if desired salifying a compound of formula (I) wherein $R^2$ is OH to provide a phytologically-acceptable salt thereof.

The following preparations and examples further illustrate the synthesis of the compounds of the present invention. It will be understood that the preparations and examples are illustrative, and that skilled organic chemists may find it advisable to modify the conditions of the following synthesis, in some instances, in order to prepare other compounds of the invention most efficiently.

## Preparation 1

### 1-(2,4-dichlorophenyl)semicarbazide

A 20 g portion of 2,4-dichlorophenylhydrazine hydrochloride was added to 200 ml of water and the solution was heated to 80°. Then 6.8 g of potassium cyanate was dissolved in 75 ml of water, and that solution was added dropwise to the first solution. The mixture was stirred for 4 hours at 80°, and it was then cooled. The product was collected by filtration, dried, and recrystallized from ethanol to obtain 11.8 g of the desired intermediate.

## Preparation 2

### 3-hydroxy-1-phenyl-1,2,4-1H-triazole

One hundred g of 1-phenylsemicarbazide was added to 500 ml of trimethyl orthoformate, and about 400 mg of p-toluenesulfonic acid was added to the mixture. It was heated on a steam bath with strong agitation for 2 hours, and was then transferred to a large flask, cooled and evaporated under vacuum. The residue was recrystallized from about 1600 ml of acetic acid to obtain 97 g of the desired intermediate, m.p. 277-280°.

Theory: C, 59.62; H, 4.38; N, 26.07;
Found: C, 59.35; H, 4.25; N, 25.86.

## Preparation 3

### 3-hydroxy-5-methyl-1-phenyl-1,2,4-1H-triazole

Twenty-five g of 1-phenylsemicarbazide and 150 ml of trimethyl orthoacetate were combined, and 100 mg of p-toluenesulfonic acid was added. The mixture was stirred on a steam bath for 5 hours, and was then cooled and evaporated under vacuum. The residue was recrystallized from ethanol to obtain 24.6 g of the desired product.

Theory: C, 61.70; H, 5.18; N, 23.99;
Found: C, 61.62; H, 5.32; N, 23.79.

## Preparation 4

### 1-(4-chlorophenyl)thiosemicarbazide

Fifty g of 4-chlorophenylhydrazine hydrochloride and 40 g of ammonium thiocyanate were added to 250 ml of denatured ethanol and the mixture was stirred under reflux for 16 hours. It was then filtered while still hot and the filter cake was washed with about 100 ml of hot ethanol. As the filtrate cooled, the product began to crystallize, and it was collected and recrystallized from denatured ethanol to obtain 32.7 g of the desired intermediate, m.p. 200-201°.

Theory: C, 41.69; H, 4.00; N, 20.84;
Found: C, 41.66; H, 3.85; N, 20.56.

Preparation 5

1-(4-chlorophenyl)-3-thiono-1,2,4-1H-triazole

Twelve g of 1-(4-chlorophenyl)thiosemicarbazide was suspended in 60 ml of 95% formic acid, and the mixture was stirred under reflux for 24 hours. The mixture was then cooled, and the product precipitated. It was collected and washed with cold denatured ethanol, and was dried to obtain 6.1 g of the desired intermediate, m.p. 222-224°.

Theory: C, 45.39; H, 2.86; N, 19.85;
Found: C, 45.41; H, 2.60; N, 19.62.

The above preparations illustrate the synthesis of the first and second intermediates for all of the products of the present invention. Only minor adjustment of the above conditions will be found to be necessary in order to maximize the efficiency of the preparation of any intermediate.

## Example 1

3-(1-ethoxycarbonylethoxy)-1-phenyl-1,2,4-1H-triazole

Five grams of the compound of Preparation 2 was dissolved in 75 ml of dimethylsulfoxide, and to it was added 1.7 g of sodium methoxide. The solution was stirred on a steam bath for 1 hour, and it was then briefly put under vacuum to remove the methanol which was formed. To it was then added 5.6 g of ethyl 2-bromopropionate, and the mixture was stirred on the steam bath overnight. It was then poured over ice-water, and the resulting solid was collected, dried and crystallized from methanol-water. The precipitate was taken up in a small amount of diethyl ether, washed with 1N aqueous sodium hydroxide and then with water, and was dried over magnesium sulfate. The solvent was removed under vacuum to obtain 600 mg of the desired product, m.p. 88-89°.

Theory: C, 59.76; H, 5.79; N, 16.08;

Found: C, 59.64; H, 5.97; N, 15.85.

## Example 2

3-(1-ethoxycarbonylethoxy)-1-(2,4-dichlorophenyl)-1,2,4-1H-triazole

Thirteen grams of 3-hydroxy-1-(2,4-dichloro phenyl)-1,2,4-1H-triazole was added to a solution of sodium methoxide (prepared from 1.3 g of sodium and excess methanol) in 200 ml of dimethylsulfoxide. The

solution was heated on a steam bath for 2 hours, and then 10 g of ethyl 2-bromopropionate was added and heating was continued for 1.5 hours more. The mixture was then cooled, poured over ice-water, and extracted with 200 ml of diethyl ether. The ether extract was washed with 1N hydrochloric acid, with saturated aqueous sodium bicarbonate, and finally with saturated brine, and was dried with sodium sulfate and phase separation paper. The solvent was removed under vacuum to obtain 16 g of the desired product, an oil.

Theory: C, 47.29; H, 3.97; N, 12.73;
Found: C, 47.09; H, 4.06; N, 12.48.

## Example 3

3-(1-ethoxycarbonylethoxy)-1-(3-trifluoromethylphenyl)-1,2,4-1H-triazole

A 1.7 g portion of sodium was dissolved in 30 ml of methanol, and the solution was added to 150 ml of dimethylsulfoxide. To it was added 17 g of 3-hydroxy-1-(3-trifluoromethylphenyl)-1,2,4-1H-triazole, and the solution was heated on the steam bath for 90 minutes. To it was then added 13.4 g of ethyl 2-bromopropionate, and heating was continued for 90 minutes more. The mixture was then cooled and poured over ice-water. The solid was collected, dried and recrystallized from ethanol to obtain 20.5 g of the desired product, m.p. 100-102°.

Theory: C, 51.07; H, 4.29; N, 12.76;
Found: C, 50.83; H, 4.48; N, 12.83.

## Example 4

1-(2-chlorophenyl)-3-(1-ethoxycarbonylethoxy)-1,2,4-1H-triazole

The process was carried out essentially according to that of Example 2 above, starting with 1.2 g of sodium, 30 ml of methanol, 10 g of 1-(2-chlorophenyl)-3-hydroxy-1,2,4-1H-triazole and 9.3 g of ethyl 2-bromopropionate. The solvent was 100 ml of dimethylsulfoxide. The product obtained was 14 g of the desired product, an oil.

Theory: C, 52.80; H, 4.77; N, 14.21;
Found: C, 52.61; H, 4.77; N, 13.98.

## Example 5

1-(3-chlorophenyl)-3-(1-ethoxycarbonylethoxy)-1,2,4-1H-triazole

A 2.2 g portion of sodium was dissolved in 75 ml of methanol and the solution was added to 100 ml of dimethylsulfoxide. To that was added 19.2 g of 1-(3-chlorophenyl)-3-hydroxy-1,2,4-1H-triazole, and the mixture was stirred on the steam bath for 4 hours. To it was then added 17.7 g of ethyl 2-bromopropionate, and heating was continued for 2 hours more. The mixture was then cooled and poured over ice-water, and the solid was collected, dried and recrystallized from ethanol and then from toluene to obtain 8.5 g of the desired product, m.p. 70-72°.

Theory: C, 52.80; H, 4.77; N, 14.21;
Found: C, 52.97; H, 4.60; N, 14.47.

Example 6

1-(3,4-dichlorophenyl)-3-(1-ethoxycarbonylethoxy)-1,2,4-1H-triazole

The process was carried out essentially like that of Example 5, using 0.8 g of sodium, 20 ml of methanol, 100 ml of dimethylsulfoxide, 8 g of 1-(3,4-dichlorophenyl)-3-hydroxy-1,2,4-1H-triazole and 6.3 g of ethyl 2-bromopropionate. The product was 7.7 g of the desired product, m.p. 69-71°.

Theory: C, 47.29; H, 3.97; N, 12.73;
Found: C, 47.13; H, 3.87; N, 12.54.

Example 7

3-(1-ethoxycarbonylethoxy)-5-methyl-1-phenyl-1,2,4-1H-triazole

A 3.2 g portion of sodium was dissolved in 50 ml of methanol, and the solution was added to 100 ml of dimethylsulfoxide. To it was added 24.6 g of 3-hydroxy-5-methyl-1-phenyl-1,2,4-1H-triazole, and the solution was heated on the steam bath for 2 hours, adding 100 ml of additional dimethylsulfoxide. To the solution was then added 25.4 g of ethyl 2-bromopropionate, and heating was continued for 2 hours more. The reaction mixture was then cooled, poured over ice-water, and extracted with 300 ml of chloroform. The extract was

washed with water and with saturated brine, and was dried with sodium sulfate and phase separation paper. The solvent was removed under vacuum, and the product was distilled at 1.2 mm of mercury and 192° to obtain 20 g of the desired product, an oil.

Theory: C, 61.08; H, 6.22; N, 15.26;
Found: C, 61.37; H, 6.34; N, 15.39.

Example 8

3-(1-ethoxycarbonylethoxy)-1-(4-methoxyphenyl)-1,2,4-1H-triazole

A 1.3 g portion of sodium was dissolved in 30 ml of methanol, and the solution was added to 150 ml of dimethylsulfoxide. To it was added 11 g of 3-hydroxy-1-(4-methoxyphenyl)-1,2,4-1H-triazole, and the solution was heated at 60° for 2 hours. To it was then added 10.4 g of ethyl 2-bromopropionate, and the mixture was heated on the steam bath for 4 hours more. It was then cooled and poured over ice-water, and the solid was collected, dried and recrystallized from ethanol-water. The impure product was purified by taking it up in 1:1 ethyl acetate:hexane and passing it through a high performance liquid chromatographic column filled with silica gel. The solvent was removed from the product-containing fractions to obtain 6.3 g of the desired product, m.p. 62-64°.

Theory: C, 57.72; H, 5.88; N, 14.42;
Found: C, 57.43; H, 5.69; N, 14.70.

## Example 9

3-(1-ethoxycarbonylbutoxy)-1-phenyl-1,2,4-1H-triazole

The synthesis was carried out substantially as shown above in Example 7, except that it was necessary to chromatograph the product as described in Example 8. The reactants were 0.7 g of sodium, 20 ml of methanol, 5 g of 3-hydroxy-1-phenyl-1,2,4-1H-triazole and 6.5 g of ethyl 2-bromopentanoate. The solvent was 80 ml of dimethylsulfoxide. Five grams of the desired product, an oil, was obtained.

Theory: C, 62.27; H, 6.62; N, 14.52;
Found: C, 62.04; H, 6.90; N, 14.25.

## Example 10

3-(1-ethoxycarbonylpentoxy)-1-phenyl-1,2,4-1H-triazole

The process was carried out as shown in Example 7 above, beginning with 1.4 g of sodium, 30 ml of methanol, 150 ml of dimethylsulfoxide, 10 g of 3-hydroxy-1-phenyl-1,2,4-1H-triazole and 13.8 g of ethyl 2-bromohexanoate. The product was distilled at 205° and 1 mm of mercury to obtain 5.2 g of the desired product, an oil.

Theory: C, 63.35; H, 6.98; N, 13.85;
Found: C, 63.62; H, 7.14; N, 13.75.

Example 11

3-(1-ethoxycarbonylethoxy)-1-(4-methylphenyl)-1,2,4-1H-triazole

The process was carried out as described in Example 8, starting with 1.1 g of sodium, 20 ml of methanol, 150 ml of dimethylsulfoxide, 8.5 g of 3-hydroxy-1-(4-methylphenyl)-1,2,4-1H-triazole and 8.7 g of ethyl 2-bromopropionate. A yield of 3.8 g of the desired product, m.p. 69-71°, was obtained.

Theory: C, 61.08; H, 6.22; N, 15.27;
Found: C, 60.90; H, 6.22; N, 15.11.

Example 12

1-(4-chlorophenyl)-3-(1-ethoxycarbonylethoxy)-1,2,4-1H-triazole

Six grams of 1-(4-chlorophenyl)-3-hydroxy-1,2,4-1H-triazole was added to a suspension of 1.5 g of sodium hydride in 225 ml of dimethylsulfoxide. The mixture was heated on the steam bath for 90 minutes. Then 5.5 g of ethyl 2-bromopropionate was added and the mixture was heated for 90 minutes more. It was then cooled and poured over ice-water, and the solid was collected, dried and recrystallized from toluene to obtain 1.5 g of the desired product, m.p. 62-64°.

Theory: C, 52.80; H, 4.77; N, 14.21;
Found: C, 52.89; H, 4.89; N, 14.08.

## Example 13

1-(4-chlorophenyl)-3-(1-ethoxycarbonylethoxy)-5-methyl-1,2,4-1H-triazole

The process was carried out as described in Example 4, except that the product was purified by chromatography on a silica gel column, eluting with 1-2 ethyl acetate-hexane. The materials were 0.5 g of sodium, 15 ml of methanol, 50 ml of dimethylsulfoxide, 4.9 g of 1-(4-chlorophenyl)-3-hydroxy-5-methyl-1,2,4-1H-triazole and 4.2 g of ethyl 2-bromopropionate. The yield was 4.5 g of the desired product, an oil.

Theory: C, 54.29; H, 5.21; N, 13.57;
Found: C, 54.04; H, 5.39; N, 13.32.

## Example 14

3-(1-ethoxycarbonylethoxy)-1-(2,6-dimethylphenyl)-1,2,4-1H-triazole

The process was carried out as described in Example 13, except that the chromatography column was eluted with 1-1 ethyl acetate-pentane. The materials were 0.8 g of sodium, 20 ml of methanol, 50 ml of dimethylsulfoxide, 6.9 g of 3-hydroxy-1-(2,6-dimethylphenyl)-1,2,4-1H-triazole and 6.6 g of ethyl 2-bromopropionate. The yield was 6 g of the desired product, an oil.

Theory: C, 62.27; H, 6.62; N, 14.52;
Found: C, 61.97; H, 6.87; N, 14.31.

Example 15

3-(1-ethoxycarbonylethoxy)-1-(3-methylphenyl)-1,2,4-1H-triazole

The process was carried out essentially as was that of Example 7, except that the first aqueous mixture was extracted with diethyl ether, rather than with chloroform. The materials were 1.5 g of sodium, 20 ml of methanol, 11.4 g of 3-hydroxy-1-(3-methylphenyl)-1,2,4-1H-triazole and 11.8 g of ethyl 2-bromopropionate. The yield was 12.9 g of the desired product, an oil.

Theory: C, 61.08; H, 6.22; N, 15.26;
Found: C, 60.83; H, 6.09; N, 15.08.

Example 16

3-(1-ethoxycarbonylpropoxy)-1-phenyl-1,2,4-1H-triazole

The process was carried out essentially as was Example 7 above, except that the aqueous mixture was extracted with ethyl acetate, rather than with chloroform, and the product was not distilled. The materials were 0.7 g of sodium, 20 ml of methanol, 50 ml of dimethylsulfoxide, 5 g of 3-hydroxy-1-phenyl-1,2,4-1H-triazole and 6 g of ethyl 2-bromobutyrate. The yield was 5.2 g of the desired product, an oil.

Theory: C, 61.08; H, 6.22; N, 15.26;
Found: C, 60.82; H, 6.37; N, 14.99.

## Example 17

1-(3-chlorophenyl)-3-(1-ethoxycarbonylethoxy)-5-methyl-1,2,4-1H-triazole

The process was carried out as was Example 9 above, starting with 0.8 g of sodium, 20 ml of methanol, 75 ml of dimethylsulfoxide, 7.8 g of 1-(3-chlorophenyl)-3-hydroxy-5-methyl-1,2,4-1H-triazole and 6.7 g of ethyl 2-bromopropionate. The yield was 7.8 g of the desired product, an oil.

Theory: C, 54.29; H, 5.21; N, 13.57;
Found: C, 53.97; H, 5.49; N, 13.80.

## Example 18

3-(1-ethoxycarbonylethoxy)-1-(2-methylphenyl)-1,2,4-1H-triazole

The process was carried out as was Example 4 above, except that the aqueous mixture was extracted with ethyl acetate. The process started with 0.7 g of sodium, 15 ml of methanol, 100 ml of dimethylsulfoxide, 5.4 g of 3-hydroxy-1-(2-methylphenyl)-1,2,4-1H-triazole and 5.6 g of ethyl 2-bromopropionate. The yield was 5.0 g of the desired product, an oil.

Theory: C, 61.08; H, 6.22; N, 15.26;
Found: C, 61.34; H, 5.97; N, 15.03.

## Example 19

1-(4-bromophenyl)-3-(1-ethoxycarbonylethoxy)-1,2,4-1H-triazole

A 3.9 g portion of sodium was dissolved in 50 ml of ethanol and was added to a solution of 41 g of 1-(4-bromophenyl)-3-hydroxy-1,2,4-1H-triazole in 150 ml of dimethylsulfoxide. The mixture was heated at 60° for 45 minutes, and then 30.9 g of ethyl 2-bromopropionate was added. The mixture was heated for 2 hours more, and was then cooled and poured over ice-water. The solid was collected and dried, and was recrystallized from toluene. The mother liquor was concentrated under vacuum, and the residue was recrystallized from ethanol. That product was combined with the first portion of product and recrystallized from ethanol to obtain 27.9 g of the desired product, m.p. 64-66°.

Theory: C, 45.90; H, 4.15; N, 12.35;
Found: C, 45.77; H, 3.96; N, 12.39.

## Example 20

1-(3-bromophenyl)-3-(1-ethoxycarbonylethoxy)-1,2,4-1H-triazole

The process was carried out as described in Example 3, starting with 2.8 g of sodium, 50 ml of ethanol, 75 ml of dimethylsulfoxide, 29 g of 1-(3-bromophenyl)-3-hydroxy-1,2,4-1H-triazole and 21.9 g of ethyl 2-bromopropionate. The yield was 31.8 g of the desired product, m.p. 83-85°.

Theory: C, 45.90; H, 4.15; N, 12.35;
Found: C, 46.11; H, 3.91; N, 12.32.

## Example 21

1-(3,4-dichlorophenyl)-3-(1-ethoxycarbonylpropoxy)-1,2,4-1H-triazole

The process was carried out as was Example 4, starting with 0.4 g of sodium, 20 ml of methanol, 75 ml of dimethylsulfoxide, 4 g of 1-(3,4-dichlorophenyl)-3-hydroxy-1,2,4-1H-triazole and 3.4 g of ethyl 2-bromobutyrate. The yield was 6 g of impure product, containing both the ethyl and methyl esters. The product was not purified but was hydrolyzed in Example 33 below.

## Example 22

3-(1-carboxyethoxy)-1-phenyl-1,2,4-1H-triazole

A 2.5 g portion of the compound of Example 1 and 2.2 g of potassium hydroxide were added to 50 ml of ethanol, and the mixture was refluxed for 3 hours. It was then poured into ice-water and the mixture was made acid with concentrated hydrochloric acid. The solid was collected and crystallized from ethanol to obtain 0.9 g of the desired product, m.p. 140-142°.

Theory: C, 56.65; H, 4.75; N, 18.02;
Found: C, 56.32; H, 4.36; N, 17.91.

Example 23

3-(1-carboxyethoxy)-1-(2,4-dichlorophenyl)-1,2,4-1H-triazole

Seven grams of the compound of Example 2 was refluxed for 3 hours with 2.4 g of potassium hydroxide in 100 ml of ethanol, and the product was collected as shown in Example 22 and recrystallized from toluene to obtain 2.3 g of the desired product, m.p. 151-153°.

Theory: C, 43.73; H, 3.00; N, 13.91;
Found: C, 43.96; H, 3.25; N, 13.69.

Example 24

3-(1-carboxyethoxy)-1-(3-trifluoromethylphenyl)-1,2,4-1H-triazole

Fifteen grams of the compound of Example 3 was refluxed for 3 hours with 5.1 g of potassium hydroxide in 150 ml of ethanol, and the product was collected as shown in Example 22 and recrystallized from ethanol to obtain 11.8 g of the desired product, m.p. 206-208°.

Theory: C, 47.85; H, 3.35; N, 13.95;
Found: C, 47.93; H, 3.52; N, 13.93.

Example 25

3-(1-carboxyethoxy)-1-(3-chlorophenyl)-1,2,4-1H-triazole

A 6.7 g portion of the compound of Example 5 was refluxed for 3 hours with 2.5 g of potassium hydroxide in 100 ml of ethanol and the product was collected as shown in Example 22 and recrystallized from ethanol to obtain 5.2 g of the desired product, m.p. 197-199°.

Theory: C, 49.36; H, 3.77; N, 15.70;
Found: C, 49.42; H, 3.69; N, 15.60.

## Example 26

3-(1-carboxyethoxy)-1-(2-chlorophenyl)-1,2,4-1H-triazole

Ten grams of the compound of Example 4 was refluxed for 4 hours with 3.8 g of potassium hydroxide in 100 ml of ethanol, and the product was collected as shown in Example 22 and recrystallized from toluene and then from ethanol to obtain 3.1 g of the desired product, m.p. 104-106°.

Theory: C, 49.36; H, 3.77; N, 15.70;
Found: C, 49.12; H, 3.79; N, 15.49.

## Example 27

3-(1-carboxyethoxy)-1-(4-chlorophenyl)-1,2,4-1H-triazole

Three grams of the compound of Example 12 was refluxed for 2 hours with 1.1 g of potassium hydroxide in 100 ml of ethanol, and the product was collected as shown in Example 22 and was recrystallized from ethyl acetate to obtain 1.9 g of the desired product, m.p. 196-198°.

Theory: C, 49.36; H, 3.77; N, 15.70;
Found: C, 49.17; H, 3.97; N, 15.46.

Example 28

3-(1-carboxyethoxy)-1-(3,4-dichlorophenyl)-1,2,4-1H-triazole

Four grams of the compound of Example 6 was refluxed for 4 hours with 1.4 g of potassium hydroxide in 75 ml of ethanol, and the product was collected as shown in Example 22 and recrystallized from ethanol to obtain 3.3 g of the desired product, m.p. 222-224°.

Theory: C, 43.73; H, 3.00; N, 13.91;
Found: C, 44.01; H, 3.01; N, 13.83.

Example 29

3-(1-carboxyethoxy)-5-methyl-1-phenyl-1,2,4-1H-triazole

Ten grams of the compound of Example 7 was refluxed for 4 hours with 4.1 g of potassium hydroxide in 150 ml of ethanol and the product was collected by extraction with ethyl acetate. The extract was washed with water, dried and evaporated under vacuum, and the residue was recrystallized from toluene to obtain 2.1 g of the desired product, m.p. 157-159°.

Theory: C, 58.29; H, 5.30; N, 16.99;
Found: C, 58.13; H, 5.32; N, 16.85.

Example 30

3-(1-carboxyethoxy)-1-(4-methoxyphenyl)-1,2,4-1H-triazole

Four grams of the compound of Example 8 was refluxed for 4 hours with 1.5 g of potassium hydroxide in 50 ml of ethanol, and the product was collected as shown in Example 22 and recrystallized from ethanol to obtain 2.6 g of the desired product, m.p. 155-157°.

Theory: C, 54.75; H, 4.98; N, 15.96;
Found: C, 54.51; H, 5.15; N, 15.75.

Example 31

3-(1-carboxyethoxy)-1-(2-methylphenyl)-1,2,4-1H-triazole

Three grams of the compound of Example 18 was refluxed for 4 hours with 1.2 g of potassium hydroxide in 100 ml of ethanol, and the product was collected as shown in Example 22 and recrystallized from ethanol to obtain 2.3 g of the desired product, m.p. 151-153°.

Theory: C, 58.29; H, 5.30; N, 16.99;
Found: C, 58.42; H, 5.45; N, 16.82.

Example 32

3-(1-carboxyethoxy)-1-(3-methylphenyl)-1,2,4-1H-triazole

Five grams of the compound of Example 15 was refluxed for 4 hours with 2 g of potassium hydroxide in

50 ml of ethanol, and the product was collected as shown in Example 22 and recrystallized from toluene to obtain 4.2 g of the desired product, m.p. 155-157°.

Theory: C, 58.29; H, 5.30; N, 16.99;
Found: C, 58.50; H, 5.35; N, 16.71.

Example 33

3-(1-carboxypropoxy)-1-(3,4-dichlorophenyl)-1,2,4-1H-triazole

Six grams of the product of Example 21 was refluxed for 2 hours with 2 g of potassium hydroxide in 100 ml of ethanol, and the acidified reaction mixture was extracted with chloroform. The extract was washed with water, dried with sodium sulfate and phase separation paper, and evaporated under vacuum. The residue was recrystallized from ethanol to obtain 3.6 g of the desired product, m.p. 232-234°.

Theory: C, 45.50; H, 3.51; N, 13.29;
Found: C, 45.85; H, 3.74; N, 13.08.

Example 34

3-(1-carboxyethoxy)-1-(2,6-dimethylphenyl)-1,2,4-1H-triazole

Three grams of the compound of Example 14 was refluxed for 3 hours with 1.2 g of potassium hydroxide in 100 ml of ethanol and the product was collected as shown in Example 22 to obtain 2.2 g of the desired product, m.p. 178-180°.

Theory: C, 59.76; H, 5.79; N, 16.08;
Found: C, 59.79; H, 6.02; N, 15.82.

## Example 35

3-(1-carboxyethoxy)-1-(4-methylphenyl)-1,2,4-1H-triazole

A 2.5 g portion of the compound of Example 11 was refluxed for 4 hours with 1 g of potassium hydroxide in 50 ml of ethanol, and the product was collected as described in Example 22 to obtain 2.0 g of the desired product, m.p. 163-165°.

Theory: C, 58.29; H, 5.30; N, 16.99;
Found: C, 58.06; H, 5.03; N, 16.79.

## Example 36

3-(1-carboxyethoxy)-1-(3-chlorophenyl)-5-methyl-1,2,4-1H-triazole

A 3.8 g portion of the compound of Example 17 was refluxed for 4 hours with 1.4 g of potassium hydroxide in 100 ml of ethanol, and the product was collected as shown in Example 22 and recrystallized from ethanol to obtain 2.8 g of the desired product, m.p. 101-103°.

Theory: C, 51.17; H, 4.29; N, 14.92;
Found: C, 51.35; H, 4.49; N, 14.84.

## Example 37

1-(4-bromophenyl)-3-(1-carboxyethoxy)-1,2,4-1H-triazole

Ten grams of the compound of Example 19 was refluxed for 10 minutes with 3.3 g of potassium hydroxide in 150 ml of ethanol, and the product was collected as described in Example 22 and recrystallized from ethanol to obtain 8.0 g of the desired product, m.p. 195-197°.

Theory: C, 42.33; H, 3.23; N, 13.46;
Found: C, 42.23; H, 3.35; N, 13.36.

## Example 38

1-(3-bromophenyl)-3-(1-carboxyethoxy)-1,2,4-1H-triazole

A 28.8 g portion of the compound of Example 20 was refluxed for 10 minutes with 9.5 g of potassium hydroxide in 100 ml of ethanol, and the product was collected as shown in Example 22 and recrystallized from ethanol to obtain 21.6 g of the desired product, m.p. 198-200°.

Theory: C, 42.33; H, 3.23; N, 13.46;
Found: C, 42.51; H, 3.45; N, 13.24.

Example 39

1-(2,4-dichlorophenyl)-3-(1-methoxycarbonylethoxy)-1,2,4-1H-triazole

Three grams of the compound of Example 23 was added to 75 ml of toluene and 5 ml of thionyl chloride, and the solution was heated under reflux for 2 hours. It was then cooled and evaporated under vacuum. Fifty ml of toluene was added to the residue, and that solvent was also removed under vacuum. To the residue was then added 50 ml of methanol, and the solution was heated under reflux for 2 hours, cooled and evaporated under vacuum. The residue was recrystallized from toluene to obtain 0.68 g of the desired product, m.p. 74-75°.

Theory: C, 45.59; H, 3.51; N, 13.29;
Found: C, 45.70; H, 3.34; N, 13.37.

Example 40

1-(2,4-dichlorophenyl)-3-(1-isopropoxycarbonylethoxy)-1,2,4-1H-triazole

Three grams of the compound of Example 23 was added to 50 ml of toluene, and 5 ml of thionyl chloride was then added. The mixture was heated under reflux for 2 hours, and was then cooled and evaporated under vacuum. Fifty ml of additional toluene was added and removed under vacuum, and then 50 ml of isopropanol was added to the residue. The mixture was heated under reflux for 2 hours, and was then cooled and evaporated under vacuum. The residue was recrystallized from isopropanol to obtain 2.6 g of the desired product, m.p. 55-57°.

Theory: C, 48.85; H, 4.39; N, 12.21;
Found: C, 48.60; H, 4.35; N, 12.00.

Example 41

3-(1-benzyloxyethoxy)-1-phenyl-1,2,4-1H-triazole

A 5.5 g portion of the compound of Example 22 was added to 50 ml of methanol containing 0.54 g of sodium, and the mixture was heated briefly to reflux. It was then cooled and the solvent was removed under vacuum. To it was added 75 ml of toluene, 2.6 ml of benzyl chloride and 2.3 g of triethylamine, and the mixture was heated under reflux for 22 hours. It was then cooled, poured over ice-water, and extracted with ethyl acetate. The extract was washed with water and with saturated brine, and was dried with sodium sulfate and phase separation paper. It was then evaporated under vacuum, and the residue was recrystallized from ethanol-water to obtain 2.5 g of the desired product, m.p. 78-80°.

Theory: C, 66.86; H, 5.30; N, 13.00;
Found: C, 66.73; H, 5.02; N, 13.07.

Example 42

3-(1-methoxycarbonylethoxy)-1-(3-trifluoromethylphenyl)-1,2,4-1H-triazole

A 2.5 g portion of the compound of Example 24 was added to 50 ml of toluene, and 3 ml of thionyl chloride was added to the mixture. It was heated under

reflux for 2 hours, and was then cooled and evaporated under vacuum. Fifty ml of toluene was added to the residue and was removed under vacuum, and the residue was then taken up in methanol and heated under reflux for 3 hours. The solvent was then removed under vacuum, and the residue was recrystallized from methanol to obtain 2.0 g of the desired product, m.p. 135-137°.

Theory: C, 49.53; H, 3.84; N, 13.33;
Found: C, 49.25; H, 3.64; N, 13.36.

Example 43

3-(1-isopropoxycarbonylethoxy)-1-(3-trifluoromethylphenyl)-1,2,4-1H-triazole

The process was carried out according to Example 42, using isopropanol instead of methanol. The product was recrystallized from toluene to obtain 0.9 g of the desired product, m.p. 97-99°.

Theory: C, 52.48; H, 4.70; N, 12.24;
Found: C, 52.27; H, 4.43; N, 12.44.

Example 44

3-(1-methylaminocarbonylethoxy)-1-phenyl-1,2,4-1H-triazole

A 2.5 g portion of the compound of Example 1 was combined with 10 ml of 40% aqueous methylamine and 20 ml of dimethylformamide at room temperature, and the mixture was allowed to stand for 2 days. It was then poured into ice-water, and the solid was collected and

air-dried to obtain 0.4 g of the desired product, m.p. 134-135°.

Theory: C, 58.53; H, 5.73; N, 22.75;
Found: C, 58.51; H, 5.82; N, 22.54.

Example 45

3-(1-dimethylaminocarbonylethoxy)-1-(3-trifluoromethylphenyl)-1,2,4-1H-triazole

Three grams of the compound of Example 24 was dissolved in 20 ml of dimethylformamide, and 3.4 g of carbonyldiimidazole was added to it. The mixture was stirred at ambient temperature for 20 minutes, and then 4 ml of 40% aqueous dimethylamine was added and the mixture was stirred at ambient temperature for 1 day. It was then poured into ice-water, and the solid was collected, dried and recrystallized from ethanol to obtain 1.5 g of the desired product, m.p. 126-127°.

Theory: C, 51.22; H, 4.61; N, 17.07;
Found: C, 50.98; H, 4.41; N, 16.99.

Example 46

3-(1-aminocarbonylethoxy)-1-(3-trifluoromethylphenyl)-1,2,4-1H-triazole

The process was carried out as was Example 45, starting with 5 g of the compound of Example 24, 4 g of carbonyldiimidazole and 1.2 g of ammonium hydroxide. The yield was 2.8 g of the desired product, m.p.

147-149°.

Theory: C, 48.01; H, 3.69; N, 18.66;
Found: C, 47.85; H, 3.63; N, 18.60.

Example 47

1-(3-chlorophenyl)-3-(1-dimethylaminocarbonylethoxy)-1,2,4-1H-triazole

The process was carried out as was Example 45, starting with 3 g of the compound of Example 25, 2.7 g of carbonyldiimidazole and 3.6 ml of 40% aqueous dimethyl-amine. The product was recrystallized from toluene to obtain 2.2 g of desired product, m.p. 136-139°.

Theory: C, 52.98; H, 5.13; N, 19.01;
Found: C, 52.93; H, 4.88; N, 18.89.

Example 48

1-(3-chlorophenyl)-3-(1-methylaminocarbonylethoxy)-1,2,4-1H-triazole

The process was carried out as was Example 45, starting with 3 g of the compound of Example 25, 2.7 g of carbonyldiimidazole, and 3 ml of 40% aqueous methylamine. The product was recrystallized from toluene to obtain 2.3 g of the desired product, m.p. 120-122°.

Theory: C, 51.35; H, 4.67; N, 19.96;
Found: C, 51.63; H, 4.89; N, 19.83.

Example 49

1-(3-chlorophenyl)-3-(1-aminocarbonylethoxy)-1,2,4-1H-triazole

The process was carried out as was Example 45, starting with 2.8 g of the compound of Example 25, 2.5 g of carbonyldiimidazole and 4 ml of ammonium hydroxide. The product was recrystallized from toluene and then from ethanol to obtain 1.3 g of the desired product, m.p. 143-145°.

Theory: C, 49.54; H, 4.16; N, 21.01;
Found: C, 49.72; H, 3.96; N, 21.20.

Example 50

3-(1-methylaminocarbonylethoxy)-1-(3-trifluoromethylphenyl)-1,2,4-1H-triazole

The process was carried out as was Example 45, starting with 3 g of the compound of Example 24, 3.4 g of carbonyldiimidazole and 3 ml of 40% aqueous methylamine. The product was not recrystallized, and amounted to 2.7 g of the desired product, m.p. 137-139°.

Theory: C, 49.96; H, 4.17; N, 17.83;
Found: C, 49.84; H, 3.94; N, 17.82.

## Example 51

3-(1-ethylaminocarbonylethoxy)-1-(3-trifluoromethylphenyl)-1,2,4-1H-triazole

The process was carried out as was Example 45, starting with 3 g of the compound of Example 24, 3.4 g of carbonyldiimidazole and 2 ml of 70% aqueous ethylamine. The product was recrystallized from ethanol to obtain 1.3 g of the desired product, m.p. 135-137°.

Theory: C, 51.22; H, 4.61; N, 17.07;
Found: C, 51.46; H, 4.62; N, 16.86.

## Example 52

3-(1-aminocarbonylethoxy)-1-(4-bromophenyl)-1,2,4-1H-triazole

The process was carried out as was Example 45, starting with 3 g of the compound of Example 37, 2.6 g of carbonyldiimidazole and 0.7 g of ammonium hydroxide. The product was recrystallized from ethanol to obtain 1.6 g of the desired product, m.p. 179-181°.

Theory: C, 42.46; H, 3.56; N, 18.01;
Found: C, 42.69; H, 3.81; N, 17.91.

## Example 53

1-(4-bromophenyl)-3-(1-methylaminocarbonylethoxy)-1,2,4-1H-triazole

The process was carried out as was Example 45, starting with 3 g of the compound of Example 37, 2.3 g

of carbonyldiimidazole and 2 ml of 40% aqueous methylamine. The product was recrystallized from ethanol to obtain 2.5 g of the desired product, m.p. 176-178°.

Theory: C, 44.33; H, 4.03; N, 17.23;
Found: C, 44.54; H, 4.23; N, 17.18.

Example 54

3-(1-aminocarbonylethoxy)-1-(3-bromophenyl)-1,2,4-1H-triazole

The process was carried out as was Example 45, starting with 3 g of the compound of Example 38, 2.3 g of carbonyldiimidazole and 0.7 g of ammonium hydroxide. A yield of 1.6 g of the desired product, in unrecrystallized form, m.p. 141-144°, was obtained.

Theory: C, 42.46; H, 3.56; N, 18.01;
Found: C, 42.70; H, 3.69; N, 17.79.

Preparation 6

3-(1-chlorocarbonylethoxy)-1-phenyl-1,2,4-1H-triazole

A 22.8 g portion of the compound of Example 22 was suspended in 150 ml of toluene, and 49 ml of thionyl chloride was added. The mixture was heated under reflux for 2 hours, and was then cooled and evaporated under vacuum. One hundred ml of additional toluene was added and was removed under vacuum, and the residue was recrystallized from toluene.

Example 55

3-(1-methoxycarbonylethoxy)-1-phenyl-1,2,4-1H-triazole

Three grams of the compound of Preparation 6 was added to 75 ml of methanol, and the solution was refluxed for an hour. It was then cooled and the solvent was removed under vacuum. The residue was taken up in 100 ml of diethyl ether, and the solution was washed with water and with saturated brine, and was dried with sodium sulfate and phase separation paper. The ether was removed under vacuum, and the residue was recrystallized from methanol to obtain 1.4 g of the desired product, m.p. 88-90°.

Theory: C, 58.29; H, 5.30; N, 16.99;
Found: C, 58.16; H, 5.11; N, 16.88.

Example 56

3-(1-isopropoxycarbonylethoxy)-1-phenyl-1,2,4-1H-triazole

Three grams of the compound of Preparation 6 was added to 75 ml of isopropanol, and the solution was heated under reflux for 19 hours. It was then cooled and evaporated under vacuum, and the residue was recrystallized from toluene to obtain 1.1 g of the desired product, m.p. 104°.

Theory: C, 61.08; H, 6.22; N, 15.26;
Found: C, 60.90; H, 5.98; N, 15.19.

Example 57

3-(1-ethoxycarbonylethoxy)-1-(3-fluorophenyl)-1,2,4-1H-triazole

A 1.7 g portion of sodium was dissolved in 50 ml of absolute ethanol, and that solution was added to a solution of 13.3 g of 1-(3-fluorophenyl)-3-hydroxy-1,2,4-1H-triazole in 150 ml of dimethylsulfoxide. The solution was heated on the steam bath for 15 minutes, and then 13.5 g of ethyl 2-bromopropionate was added and the mixture was held on the steam bath for 2 hours. It was then poured over ice-water, and the aqueous mixture was extracted with diethyl ether. The organic layer was washed with water and with brine, and was then dried with sodium sulfate and phase separation paper. The solvent was removed under vacuum, and the product was recrystallized from aqueous ethanol to obtain 6.7 g of the desired product, m.p. 35-37°.

Theory: C, 55.91; H, 5.05; N, 15.05;
Found: C, 55.68; H, 5.03; N, 14.82.

Example 58

3-(1-carboxyethoxy)-1-(3-fluorophenyl)-1,2,4-1H-triazole

Four grams of the product of Example 57 was combined with 1.6 g of potassium hydroxide in 100 ml of ethanol, and the mixture was heated briefly to reflux. It was then cooled, poured over ice-water, and made acid with hydrochloric acid. The product was collected by

filtration and dried to obtain 3.4 g of the desired compound, m.p. 177-179°.

Theory: C, 52.59; H, 4.01; N, 16.73;
Found: C, 52.76; H, 4.25; N, 16.89.

Example 59

3-(1-aminocarbonylethoxy)-1-(3-fluorophenyl)-1,2,4-1H-triazole

Two grams of the product of Example 58 was dissolved in 10 ml of dimethylformamide and 1.9 g of carbonyldiimidazole was added. The mixture was stirred at room temperature for 20 minutes, and 0.6 g of 35% ammonium hydroxide was added. The mixture was then stirred for 20 hours at room temperature and was poured over ice-water. The product was collected by filtration and dried to obtain 1.4 g of the desired product, m.p. 156-158°.

Theory: C, 52.80; H, 4.43; N, 22.39;
Found: C, 52.50; H, 4.22; N, 22.46.

Example 60

1-(4-bromophenyl)-3-(1-ethoxycarbonylethoxy)-1,2,4-1H-triazole

A 41 g portion of 1-(4-bromophenyl)-3-hydroxy-1,2,4-1H-triazole was dissolved in 150 ml of dimethylsulfoxide, and to it was added 3.9 g of sodium dissolved in 50 ml of absolute ethanol. The mixture was

stirred at 60° for 45 minutes, and 30.9 g of ethyl 2-bromopropionate was added. The mixture was stirred for 2 hours more at the same temperature. It was then cooled and poured over ice-water, and the product was collected by filtration and dried. It was recrystallized from toluene and the mother liquor was concentrated under vacuum. The residue was recrystallized from ethanol, and all of the product was then combined and recrystallized from ethanol again to obtain 27.9 g of the desired product, m.p. 64-66°.

Theory: C, 45.90; H, 4.15; N, 12.35;
Found: C, 45.77; H, 3.96; N, 12.39.

## Example 61

1-(4-bromophenyl)-3-(1-carboxyethoxy)-1,2,4-1H-triazole

Ten grams of the product of Example 60 was hydrolyzed with potassium hydroxide as shown in Example 58 to obtain 8.0 g of the desired product, m.p. 195-197°.

Theory: C, 42.33; H, 3.23; N, 13.46;
Found: C, 42.23; H, 3.35; N, 13.36.

Example 62

1-(4-bromophenyl)-3-(1-cyclopropylaminocarbonylethoxy)-1,2,4-1H-triazole

The process was carried out according to Example 59, starting with 3 g of the product of Example 61, 2.4 g of carbonyldiimidazole and 1.1 g of cyclopropylamine. The product was recrystallized from ethanol to obtain 2.6 g of the desired product, m.p. 170-172°.

Theory: C, 47.88; H, 4.31; N, 15.95;
Found: C, 47.69; H, 4.05; N, 16.19.

Example 63

1-(4-bromophenyl)-3-(1-propylaminocarbonylethoxy)-1,2,4-1H-triazole

A 2.4 g portion of carbonyldiimidazole was added to 3 g of the product of Example 61 dissolved in 20 ml of dimethylformamide and the mixture was stirred at room temperature for 20 minutes. To it was then added 1.2 g of propylamine and the mixture was stirred at room temperature for 3 days. It was then poured over ice-water, and the product was collected by filtration and dried. It was recrystallized from ethanol to obtain 2.3 g of the desired product, m.p. 144-145°.

Theory: C, 47.61; H, 4.85; N, 15.86;
Found: C, 47.85; H, 5.03; N, 15.86.

Example 64

1-(3-bromophenyl)-3-(1-ethoxycarbonylethoxy)-1,2,4-1H-triazole

A 2.8 g portion of sodium was dissolved in 50 ml of absolute ethanol and added to 29 g of 1-(3-bromophenyl)-3-hydroxy-1,2,4-1H-triazole in 75 ml of dimethylsulfoxide. The solution was heated on the steam bath for 30 minutes, and then 21.9 g of ethyl 2-bromopropionate was added and the mixture was held on the steam bath for 2 hours more. It was then cooled and poured over ice-water, and the product was collected by filtration, dried and recrystallized from ethanol to obtain 31.8 g of the desired compound, m.p. 83-85°.

Theory: C, 45.90; H, 4.15; N, 12.35;
Found: C, 46.11; H, 3.91; N, 12.32.

Example 65

1-(3-bromophenyl)-3-(1-carboxyethoxy)-1,2,4-1H-triazole

A 28.8 g portion of the product of Example 64 was hydrolyzed in ethanol with 9.5 g of potassium hydroxide, as described in Example 59. The product was recrystallized from ethanol to obtain 21.6 g of the desired product, m.p. 198-200°.

Theory: C, 42.33; H, 3.23; N, 13.46;
Found: C, 42.51; H, 3.45; N, 13.24.

Example 66

1-(3-bromophenyl)-3-(1-cyclopropylaminocarbonyl-ethoxy)-1,2,4-1H-triazole

Three grams of the product of Example 65 was dissolved in 20 ml of dimethylformamide, 2.4 g of carbonyldiimidazole was added, and the solution was stirred at room temperature for 20 minutes. To it was added 1.1 g of cyclopropylamine, and the mixture was stirred for 16 hours more. It was then poured over ice-water, and the product was collected, dried and recrystallized from ethanol to obtain 2.1 g of the desired product, m.p. 134-135°.

Theory: C, 47.88; H, 4.31; N, 15.95;
Found: C, 48.01; H, 4.25; N, 15.98.

Example 67

1-(3-bromophenyl)-3-(1-propylaminocarbonyl-±ethoxy)-1,2,4-1H-triazole

Three grams of the product of Example 65 was reacted with 1.2 g of propylamine, substantially as shown in Example 66, to obtain 2.4 g of the desired product, m.p. 111-113°.

Theory: C, 47.61; H, 4.85; N, 15.86;
Found: C, 47.42; H, 4.58; N, 15.67.

Example 68

3-(1-ethoxycarbonylethoxy)-1-(4-trifluoromethylphenyl)-1,2,4-1H-triazole

A 0.54 g portion of sodium was dissolved in 15 ml of absolute ethanol, and was added to 50 ml of dimethylsulfoxide containing 5.4 g of 3-hydroxy-1-(4-trifluoromethylphenyl)-1,2,4-1H-triazole. An additional 25 ml of dimethylsulfoxide was added, and the mixture was heated on the steam bath for 15 minutes. Then 4.3 g of ethyl 2-bromopropionate was added and heating was continued for 1 hour. The mixture was then cooled and poured over ice-water, and the product was collected, dried, and purified by high-performance liquid chromatography, eluting with 1:1 hexane:ethyl acetate. The product-containing fractions were combined and evaporated under vacuum to obtain 3.5 g of the desired product.

Theory: C, 51.07; H, 4.29; N, 12.76;
Found: C, 51.27; H, 4.42; N, 12.85.

Example 69

3-(1-carboxyethoxy)-1-(4-trifluoromethylphenyl)-1,2,4-1H-triazole

A 17.1 g portion of the compound of Example 68, prepared in successive reactions, was hydrolyzed with 5.8 g of potassium hydroxide, substantially as shown in Example 58, to obtain 15.5 g of the desired product, m.p. 215-218°.

Theory: C, 47.85; H, 3.35; N, 13.95;
Found: C, 48.04; H, 3.27; N, 13.95.

## Example 70

3-(1-aminocarbonylethoxy)-1-(4-trifluoromethylphenyl)-1,2,4-1H-triazole

Three grams of the product of Example 69 was reacted with 0.7 g of 35% ammonium hydroxide, substantially as shown in Example 62, to obtain 2 g of the desired product, m.p. 178-180°.

Theory: C, 48.01; H, 3.69; N, 18.66;
Found: C, 47.80; H, 3.52; N, 18.54.

## Example 71

3-(1-dimethylaminocarbonylethoxy)-1-(4-trifluoromethylphenyl)-1,2,4-1H-triazole

Three grams of the product of Example 69 was reacted with 4 ml of 45% aqueous dimethylamine, substantially as shown in Example 62, to obtain 1.2 g of the desired product, m.p. 102-104°.

Theory: C, 51.22; H, 4.61; N, 17.07;
Found: C, 51.04; H, 4.67; N, 17.10.

## Example 72

3-(1-cyclopropylaminocarbonylethoxy)-1-(4-trifluoromethylphenyl)-1,2,4-1H-triazole

Three grams of the product of Example 69 was reacted with 1.1 g of cyclopropylamine, substantially as shown in Example 62, to obtain 2.6 g of the desired

product, m.p. 175-177°.

Theory: C, 52.94; H, 4.44; N, 16.46;
Found: C, 52.73; H, 4.26; N, 16.60.

Example 73

3-(1-methylaminocarbonylethoxy)-1-(4-trifluoromethylphenyl)-1,2,4-1H-triazole

Three grams of the product of Example 69 was reacted with 2 ml of aqueous methylamine, substantially as shown in Example 62, to obtain 2.3 g of the desired product, m.p. 157-158°.

Theory: C, 49.69; H, 4.17; N, 17.83;
Found: C, 49.64; H, 4.23; N, 18.04.

Example 74

3-(1-ethoxycarbonylethylthio)-1-phenyl-1,2,4-1H-triazole

Three grams of 1-phenyl-3-thiono-1,2,4-1H-triazole was suspended in 50 ml of ethanol, and to it was added 0.8 g of sodium hydroxide in 30 ml of ethanol. The mixture was heated to reflux for 15 minutes, and then 3 g of ethyl 2-bromopropionate was added and the mixture was held at reflux for 2 hours. It was then cooled, poured over ice-water, and extracted with ethyl acetate. The organic layer was washed with water, with 2N aqueous sodium hydroxide and with brine, and was dried with sodium sulfate and phase separation paper. The solvent was removed under vacuum, and the residue

was purified by high-performance liquid chromatography, with 4:1 hexane:ethyl acetate as the solvent. The product-containing fractions were combined and evaporated under vacuum to obtain 1.8 g of the desired product.

Theory: C, 56.30; H, 5.45; N, 15.15;
Found: C, 56.60; H, 5.23; N, 15.34.

## Example 75

1-(3,4-dichlorophenyl)-3-(1-ethoxycarbonylethylthio)-1,2,4-1H-triazole

An 11.5 g portion of 1-(3,4-dichlorophenyl)-3-thiono-1,2,4-1H-triazole was dissolved in 100 ml of dry dimethylsulfoxide, and was added to 1.1 g of sodium dissolved in 50 ml of absolute ethanol. The mixture was warmed at 60-80° for 1 hour, and then 8.5 g of ethyl 2-bromopropionate was added and the mixture was held at 100-110° for 2 hours. It was then cooled and poured over ice-water, and the aqueous mixture was extracted with dichloromethane. The organic layer was washed with brine, and was dried over magnesium sulfate. The solvent was removed under vacuum, and the residue was purified by chromatography, eluting with 2:1 hexane:ethyl acetate. The product-containing fractions were combined and evaporated under vacuum to obtain 11 g of the desired product.

Theory: C, 45.10; H, 3.78; N, 12.14;
Found: C, 44.91; H, 3.72; N, 11.87.

Example 76

3-(1-ethoxycarbonylethoxy)-1-(4-trifluoromethoxyphenyl)-1,2,4-1H-triazole

Eleven g of 3-hydroxy-1-(4-trifluoromethoxyphenyl)-1,2,4-1H-triazole was dissolved in 100 ml of dimethylsulfoxide, and to it was added a solution of sodium methoxide prepared from 1.2 g of sodium and 20 ml of absolute ethanol. An additional 100 ml of dimethylsulfoxide was added, and the solution was heated on the steam bath for 90 minutes. Then 8.3 g of ethyl 2-bromopropionate was added, and heating was continued for 90 minutes more. The solution was cooled and poured over ice water, and the solid was collected and dried to obtain 15 g of the desired product, m.p. 52-53°.

Theory: C, 48.70; H, 4.09; N, 12.17;
Found: C, 48.93; H, 4.21; N, 12.34.

Example 77

3-(1-carboxyethoxy)-1-(4-trifluoromethoxyphenyl)-1,2,4-1H-triazole

Thirteen g of the compound of Example 76 was deesterified according to the process of Example 22, and the product was isolated and purified, following the same Example, to obtain 6.8 g of the desired product, m.p. 200-203°.

Theory: C, 45.44; H, 3.18; N, 13.25;
Found: C, 45.20; H, 3.16; N, 13.08.

Example 78

1-(3,5-dichlorophenyl)-3-(1-ethoxycarbonylethoxy)-1,2,4-1H-triazole

A 1.8 g portion of sodium was dissolved in 75 ml of absolute ethanol, and the solution was added slowly with stirring to a solution of 18.5 g of 1-(3,5-dichlorophenyl)-3-hydroxy-1,2,4-1H-triazole in 200 ml of dimethylsulfoxide. The solution was stirred at 60° for 1 hour, 14.5 g of ethyl 2-bromopropionate was added, and the mixture was heated for 2 hours at 100-110°. It was then poured over ice water, and the solid was collected, dried and recrystallized from ether to obtain 10 g of the desired product. The mother liquor was chromatographed to obtain 1.3 g of additional product.

Theory: C, 47.29; H, 3.97; N, 12.73;
Found: C, 47.07; H, 4.03; N, 12.57.

Example 79

3-(1-carboxyethoxy)-1-(3,5-dichlorophenyl)-1,2,4-1H-triazole

An 11.3 g portion of the product of Example 78 was deesterified according to the process of Example 22, and was recrystallized from ethanol/dichloromethane to obtain 10.5 g of the desired product, m.p. 230-231°.

Theory: C, 43.73; H, 3.00; N, 13.91;
Found: C, 44.08; H, 3.24; N, 13.63.

(this page follows page 53B)

Methods of Use

The compounds of the present invention have a variety of regulatory effects on the growth habits of numerous species of plants. Perhaps the most important effect of the compounds is their ability to slow the growth and decrease the size of plants. Thus, the treated plants occupy less space and can be planted more densely. The treated plants are shorter and stronger, and therefore less likely to be lodged by wind and rain. In general, the smaller plant will have a reduced need for nutrients, as well.

The plants also exhibit effects on the pollen-producing and seed-producing organs of plants, particularly of monocotyledons such as grasses, wheat, rice, corn and the like, but also on dicotyledons such as cotton and the like. Thus, when a compound of the invention is applied to a turf grass, the formation of seed heads as well as the growth of the grass is inhibited. The appearance of the turf is greatly improved thereby. Further, and of more economic importance, is the gametocidal effect of the compounds. Application of a compound to a plant will inhibit or prevent the plants' production of pollen. Thus, the non-pollenating plant can be easily used as the female parent in plant hybridization. For example, the compounds can beneficially be used as gametocides in producing hybrid corn, wheat, rice and the like.

Further, the compounds increase branching, flowering, and the number of pods set on various crop plants, particularly on soybeans. As a result, the yield per plant is increased over untreated plants. Since it may well be possible to plant more closely when

a compound of the invention is to be used, the possibility of significant yield benefits is offered.

Still further, the compounds confer other growth effects. Depending on the species of plant of concern, the application rate, the time of application and perhaps other factors as well, effects such as reduced internodal length, increased number of nodes, an increase in the number of nodes which bear a leaf, a branch or a flower, improvement in the plant's or an individual leaf's morphology, or improvements in the structure and extent of the treated plant's root system, are provided by compounds of the invention.

The soybean is a particularly preferred plant for use of the present compounds. A group of preferred plants includes soybean, dry bean and other beans grown for human consumption, alfalfa, cotton and peanut. Another group of preferred plants includes turf grasses, corn, wheat, barley, rice, rye and oats. Still another group of preferred plants includes fruit trees, ornamental trees and nut trees. A group of more highly preferred plants for use of the present compounds includes soybean, sugar beet, cotton and corn.

The time and manner of applying a compound of the present invention to a plant may be varied. It appears to be preferable to apply it before or near the beginning of the plant's reproductive stage. The compounds may be beneficially applied, however, up to the late reproductive stage of the plant.

It is preferred to apply the compound directly to the plant, or to the soil in which the plant is rooted, as the plant nears the stage at which it is to be treated. It is most preferred to apply the compound as a foliar spray directly to the plant. It is conventional to formulate agriculturally useful compounds as water-dispersed formulations for such applications, and the use of such compositions is preferred in the practice of the present invention.

It may be more beneficial to apply a compound two or more times at relatively short intervals. Plants in a field do not all develop at the same time, even though they were all planted at once. Therefore, an application which is perfectly timed for the slower-developing plants may be too late for the earlier ones. Applying a compound twice, or even three or four times, at intervals of a few or several days will improve its performance, and therefore the performance of the treated crop, under some circumstances.

Many of the compounds are active at extremely low application rates. A preferred range of application rates is from about 0.015 to about 0.5 lb/A. Another preferred range of rates, particularly for use on soybeans, is from about 0.06 to about 0.25 lb/A. From about 0.03 to about 0.25 lb/A is another preferred rate range. Still another preferred range, particularly for the less active compounds, is from about 0.25 to about 2 lb/A. However, rates in the broad range from about 0.015 to about 5 lb/A are used in particular instances, particularly when the maximum degree of size reduction, or other regulatory effect, is called for.

## Compositions

Formulated compositions of the compounds of the present invention constitute an important aspect of the invention. Such compositions comprise a compound of the invention and a phytologically-acceptable diluent. The most economical and preferred compositions are concentrated water-emulsifiable or water-dispersable compositions. Such compositions include, in general, emulsifiable concentrates, suspension concentrates, wettable powders and wettable granules, all of which are commonly used in the agricultural chemical art. Some discussion of them will be provided, however, to assure understanding by the reader.

The concentration of a compound in a concentrated composition is entirely irrelevant to the use of the compound. Such compositions are diluted in water for application, and the application rate of the compound is determined by the ratio at which the composition is diluted in water, or by the amount of the composition which is applied per area of crop land. Thus, any desired application rate can be obtained from any concentrated composition. Farmers and agricultural chemists are acquainted with the simple calculations which are necessary.

Emulsifiable concentrates of the compounds comprise a convenient concentration of the compound dissolved in a phytologically-acceptable diluent which is a mixture of water-immiscible organic solvent and emulsifiers. Useful organic solvents, in general, include aromatics, especially xylenes, and the petroleum

fractions, especially the naphthalenic and olefinic portions of petroleum, such as those called heavy aromatic naphthas. Terpenic solvents including rosin derivatives, and complex alcohols such as 2-ethoxyethanol are also often used, and amides such as dimethyl-acetamide may be particularly useful with the present compounds. Suitable emulsifiers for emulsifiable concentrates, generally used in amounts in the range of from about 1% to about 10% by weight of the concentrate, are frequently found among the alkylbenzenesulfonates, the alkyl sulfates, the non-ionics such as ethylene oxide adducts of alkylphenol, and especially among the metal and amine salts of alkyl sulfates.

Wettable powders comprise an intimate mixture of the compound and a phytologically-acceptable diluent made up of an inert carrier and surfactants. The inert carrier is usually chosen from among easily water-dispersable powdery substances such as attapulgite clay, the montmorillonite clays, the diatomaceous earths and the purified silicates. Surfactants for wettable powders are found among the same types just mentioned for emulsifiable concentrates, as well as the sulfonated lignins and the naphthalenesulfonates. It is possible to compact a wettable powder into granular form, and thereby to produce a wettable granule, which has the advantage of being non-dusty and easy to measure and pour. When added to water, a properly formulated wettable granular product will disperse and become a fine suspension.

The compounds may also be formulated as suspensions, which consist of a relatively high concentration, in the interest of economy, of the compound in finely powdered form, dispersed and suspended in a phytologically-acceptable aqueous diluent. A surfactant system for a suspension product is much like that used in a wettable powder, but it must be capable of maintaining the compound in dispersed form over a long period of time. It is sometimes advisable to adjust the density of the liquid, as by dissolving an inert salt in it, in order to assist in the suspension of the relatively dense particles of compound.

When an aqueous dispersion of a compound, prepared by the dilution of a concentrated composition, is to be applied to foliage, an adjuvant is often used to improve the ability of the dispersion to wet and adhere to the foliage. Such adjuvants as vegetable gums, emulsified polybutenes, cationic and other surfactants and lignin derivatives are often used. The use of an adjuvant in aqueous dispersions of the present compounds is highly preferred, and regularly improves results. Not only the commercial adjuvants, which are commonly known to growers, but also ordinary surfactants, are beneficially used, at concentrations in the range of a few tenths of a percent in the dispersion.

Aqueous dispersions of concentrated compositions may be applied either to foliage or to the soil in which the plants grow. When the application is to be to the soil, a granular composition can also be effectively used. A granular agricultural composition consists of the compound, applied, usually in a relatively low

concentration such as from about 0.1% to about 10% by weight, to a granular carrier having a convenient particle size for application. Typically, the particle size range is from 20 to 60 mesh, on the standard U.S. sieve size scale. Such carriers as clay, sand, pulverized stone, corncob grits and the like are frequently used and may be chosen for convenience and economy. It is usually unnecessary to use any adjuvant or other ingredient other than the compound and the carrier, with perhaps a small amount of solvent in which the compound is applied to the carrier. The carrier may also be supplied in powdered form, and formulated by mixing the powdered carrier with the powdered compound and then compacting the mixture and granulating it to the desired particle size range.

The following examples of compositions of compounds of the present invention are given, to assure that agricultural chemists understand the manner in which the compounds are formulated.

Suspension of Example 1

| | |
|---|---|
| Compound of Example 1 | 12.5% |
| Tergitol TMN-6 (non-ionic surfactant) | 10.0 |
| Purified silica | 1.0 |
| Polyfon H (lignin sulfonate salt) | 0.5 |
| 2% xanthan gum | 10.0 |
| Silicone antifoam | 0.2 |
| Water | 65.8 |

The compound was ground with the silica, the Polyfon and part of the water in an attrition mill until 50% of the particles were smaller than 2.3 microns. The milled suspension was then mixed with the rest of the ingredients.

Wettable Powder of Example 22

| | |
|---|---|
| Compound of Example 22 | 52.1% |
| Polyfon O (lignin sulfonate salt) | 5.0 |
| Purified silica | 5.0 |
| Sodium lauryl sulfate | 5.0 |
| Kaolin clay | 32.9 |

The ingredients were mixed and milled twice through an air impact mill, so that 50% of the particles were smaller than 4.2 microns.

Suspension of Example 22

| | |
|---|---|
| Compound of Example 22 | 12.5% |
| Purified silica | 1.0 |
| Polyfon H | 0.5 |
| Silicone antifoam | 0.2 |
| Tergitol TMN-6 | 10.0 |
| 2% xanthan gum | 10.0 |
| Water | 65.8 |

The compound was milled with the Tergitol, the antifoam, the silica, the Polyfon and part of the water in an attrition mill until 50% of the particles were

smaller than 2 microns. It was then mixed with the xanthan and the rest of the water.

Suspension of Example 5

| | |
|---|---|
| Compound of Example 5 | 12.5% |
| Silicone antifoam | 0.2 |
| Purified silica | 1.0 |
| 2% xanthan gum | 10.0 |
| Nonionic surfactant | 10.0 |
| Water | 66.3 |

The product was processed as was the suspension immediately above.

Emulsifiable Concentrate of Example 1

| | |
|---|---|
| Compound of Example 1 | 6.2% |
| Propylene glycol methyl ether | 20.0 |
| Heavy aromatic naphtha | 70.6 |
| Toximul H (anionic surfactant) | 1.6 |
| Toximul D (anionic surfactant) | 1.6 |

Suspension of Example 3

| | |
|---|---|
| Compound of Example 3 | 12.5% |
| Inert ingredients as used in Suspension of Example 5 | 87.5 |

The product was processed as was the Suspension of Example 5, milling to a particle size range where 50% of the particles were smaller than 2.2 microns.

Suspension of Example 6

| | |
|---|---|
| Compound of Example 6 | 12.5% |
| Inert ingredients as used in Suspension of Example 5 | 87.5 |

The product was processed as was the Suspension of Example 5, milling to a particle size range where 50% of the particles were smaller than 1.9 microns.

Emulsifiable Concentrate of Example 1

| | |
|---|---|
| Compound of Example 1 | 6.2% |
| Propylene glycol methyl ether | 20.0 |
| Heavy aromatic naptha | 68.0 |
| Toximul H | 2.9 |
| Toximul D | 2.9 |

CLAIMS

1. A compound of the formula (I)

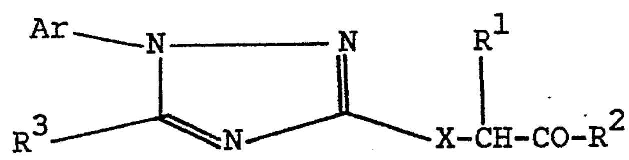

(I)

wherein

X is -O- or -S-;

Ar is phenyl or phenyl substituted with 1 or 2 groups independently selected from halo, trifluoromethyl, $C_1$-$C_3$ alkyl, $C_1$-$C_3$ alkoxy, difluromethoxy, trifluoromethoxy, pentafluoroethoxy, and 1,1,2,2-tetrafluoroethoxy;

$R^1$ is $C_1$-$C_4$ primary or secondary alkyl;

$R^2$ is hydroxy, $C_1$-$C_4$ alkoxy, benzyloxy, phenoxy, or $-NR^4R^5$;

$R^3$ is hydrogen or $C_1$-$C_4$ primary or secondary alkyl;

$R^4$ and $R^5$ independently are hydrogen, $C_3$-$C_6$ cycloalkyl or $C_1$-$C_3$ alkyl, or $R^4$ and $R^5$ combine with the nitrogen atom to which they are attached to form morpholino, pyrrolidino or piperidino; or a phytologically-acceptable salt thereof.

2. A compound of claim 1 wherein X is -O-, $R^1$ methyl, $R^2$ is $C_1$-$C_4$ alkoxy, and $R^3$ is hydrogen.

3. A compound of claim 1 wherein X is -O-, Ar is 3-chlorophenyl, 4-chlorophenyl, or 3,4-dichloro-

phenyl, $R^1$is methyl, $R^2$is $C_1$-$C_4$ alkoxy, and $R^3$ is hydrogen.

4. A compound of claim 1 wherein X is -0-, Ar is 3-chlorophenyl, 4-chlorophenyl, or 3,4-dichlorophenyl, $R^1$ is methyl, $R^2$ is OH, and $R^3$is hydrogen.

5. A method of regulating the growth of plants which comprises applying an effective amount of a compound of formula (I), as defined in claim 1, to a plant at a time not later than the late reproductive growth stage.

6. The method of claim 5 wherein the compound of formula (I) is one where X is O, $R^1$ is methyl, $R^2$ is OH or $C_1$-$C_4$ alkoxy, $R^3$ is hydrogen, and Ar is 3-chlorophenyl, 4-chlorophenyl, or 3,4-dichlorophenyl, or a phytologically acceptable salt thereof.

7. An agricultural composition which comprises a compound of formula (I) as defined in claim 1 in combination with a phytologically-acceptable diluent.

8. A composition of claim 7 wherein the compound of formula (I) is one wherein X is O, $R^1$ is methyl, $R^2$ is OH or $C_1$-$C_4$ alkoxy, $R^3$ is hydrogen and Ar is 3-chlorophenyl, 4-chlorophenyl, or 3,4-dichlorophenyl, or a phytologically acceptable salt thereof.

9. A process for preparing a compound of Formula (I) as defined in claim 1 which comprises:

a) reacting a compound of formula (II)

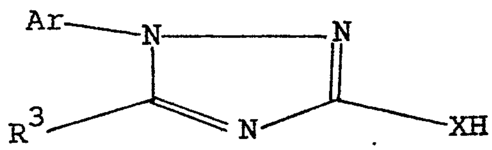 (II)

wherein Ar, $R^3$, and X are as defined above, with a compound of the formula (III)

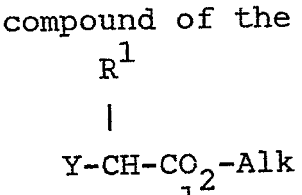 (III)

wherein $R^1$ is as defined above, Y is chloro or bromo, and Alk is $C_1$-$C_4$ alkyl, in the presence of a strong base, to produce a compound of formula (I) wherein $R^2$ is $C_1$-$C_4$ alkoxy: or

b) hydrolyzing a compound of formula (I) wherein $R^2$ is $C_1$-$C_4$ alkoxy to provide a compound of formula (I) wherein $R^2$ is OH; or

c) reacting a compound of formula (IV)

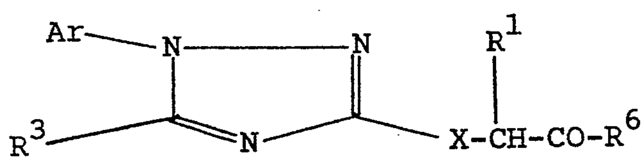 (IV)

wherein Ar, $R^1$, $R^3$, and X are as defined above and $R^6$ is OH or Cl with a $C_1$-$C_4$ alcohol to provide a compound of formula (I) wherein $R^2$ is $C_1$-$C_4$ alkoxy; or

d) reacting a compound of formula (I) wherein $R^2$ is OH or $C_1$-$C_4$ alkoxy with ammonium hydroxide or an amine of the formula $NHR^4R^5$ where $R^4$ and $R^5$ are as defined above, to produce a compound of formula (I) wherein $R^2$ is $NR^4R^5$; and

if desired salifying a compound of formula (I) wherein $R^2$ is OH to provide a phytologically-acceptable salt thereof.

0227284
AUSTRIA/SPAIN



CLAIMS

1. A process for preparing a compound of the formula (I)

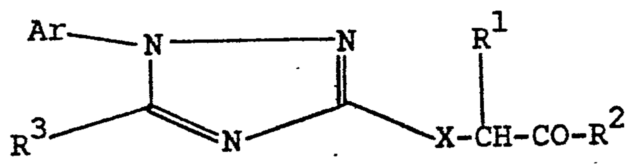

(I)

wherein

X is -O- or -S-;

Ar is phenyl or phenyl substituted with 1 or 2 groups independently selected from halo, trifluoromethyl, $C_1$-$C_3$ alkyl, $C_1$-$C_3$ alkoxy, difluromethoxy, trifluoromethoxy, pentafluoroethoxy, and 1,1,2,2-tetrafluoroethoxy;

$R^1$ is $C_1$-$C_4$ primary or secondary alkyl;

$R^2$ is hydroxy, $C_1$-$C_4$ alkoxy, benzyloxy, phenoxy, or $-NR^4R^5$;

$R^3$ is hydrogen or $C_1$-$C_4$ primary or secondary alkyl;

$R^4$ and $R^5$ independently are hydrogen, $C_3$-$C_6$ cycloalkyl or $C_1$-$C_3$ alkyl, or $R^4$ and $R^5$ combine with the nitrogen atom to which they are attached to form morpholino, pyrrolidino or piperidino; or a phytologically-acceptable salt thereof, which comprises:

a) reacting a compound of formula (II)

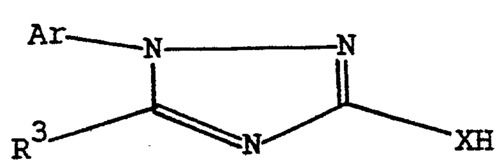

(II)

wherein Ar, $R^3$, and X are as defined above, with a compound of the formula (III)

$R^1$
|
$Y-CH-CO_2-Alk$ (III)

wherein $R^1$ is as defined above, Y is chloro or bromo, and Alk is $C_1$-$C_4$ alkyl, in the presence of a strong base, to produce a compound of formula (I) wherein $R^2$ is $C_1$-$C_4$ alkoxy: or

b) hydrolyzing a compound of formula (I) wherein $R^2$ is $C_1$-$C_4$ alkoxy to provide a compound of formula (I) wherein $R^2$ is OH; or

c) reacting a compound of formula (IV)

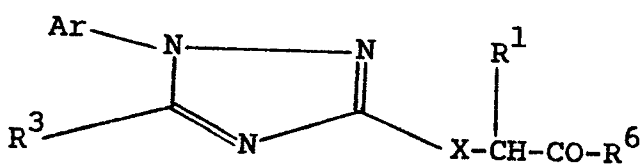
(IV)

wherein Ar, $R^1$, $R^3$, and X are as defined above and $R^6$ is OH or Cl with a $C_1$-$C_4$ alcohol to provide a compound of formula (I) wherein $R^2$ is $C_1$-$C_4$ alkoxy; or

d) reacting a compound of formula (I) wherein $R^2$ is OH or $C_1$-$C_4$ alkoxy with ammonium hydroxide or an amine of the formula $NHR^4R^5$ where $R^4$ and $R^5$ are as defined above, to produce a compound of formula (I) wherein $R^2$is $NR^4R^5$; and

if desired salifying a compound of formula (I) wherein $R^2$is OH to provide a phytologically-acceptable salt thereof.

2. The process of claim 1 wherein a compound of formula (II) in which X is O and $R^3$ is hydrogen is reacted with a compound of formula (III) in which $R^1$ is methyl to produce a compound of formula (I) wherein X is O, $R^1$ is methyl, $R^2$ is $C_1$-$C_4$ alkoxy, and $R^3$ is hydrogen.

3. The process of claim 1 wherein a compound of formula (II) in which X is O, $R^3$ is hydrogen, and Ar is 3-chlorophenyl, 4-chlorophenyl, or 3,4-dichlorophenyl, is reacted with a compound of formula (III) in which $R^1$ is methyl to produce a compound of formula (I) wherein X is O, Ar is 3-chlorophenyl, 4-chlorophenyl, or 3,4-dichlorophenyl, $R^1$ is methyl, $R^2$ is $C_1$-$C_4$ alkoxy, and $R^3$ is hydrogen.

4. The process of claim 1 wherein a compound of formula (I) in which X is O, $R^1$ is methyl, $R^2$ is $C_1$-$C_4$ alkoxy, $R^3$ is hydrogen, and Ar is 3-chlorophenyl, 4-chlorophenyl, or 3,4-dichlorophenyl is hydrolyzed to provide a compound of formula (I) wherein X is O, $R^1$ is methyl, $R^2$ is OH, $R^3$ is hydrogen, and Ar is 3-chlorophenyl, 4-chlorophenyl, or 3,4-dichlorophenyl.

5. A method of regulating the growth of plants which comprises applying an effective amount of a compound of formula (I), as defined in claim 1, to a plant at a time not later than the late reproductive growth stage.

6. The method of claim 5 wherein the compound of formula (I) is one where X is O, $R^1$ is methyl, $R^2$ is OH or $C_1$-$C_4$ alkoxy, $R^3$ is hydrogen, and Ar is 3-chlorophenyl, 4-chlorophenyl, or 3,4-dichlorophenyl, or a phytologically acceptable salt thereof.

7. An agricultural composition which comprises a compound of formula (I) as defined in claim 1 in combination with a phytologically-acceptable diluent.

8. A composition of claim 7 wherein the compound of formula (I) is one wherein X is O, $R^1$ is methyl, $R^2$ is OH or $C_1$-$C_4$ alkoxy, $R^3$ is hydrogen and Ar is 3-chlorophenyl, 4-chlorophenyl, or 3,4-dichlorophenyl, or a phytologically acceptable salt thereof.

European Patent Office

# EUROPEAN SEARCH REPORT

0227284
Application number

EP 86 30 8759

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 155 486 (ALKALOIDA VEGYéSZETIGYáR) * page 7, line 24 - page 8, line 13; examples 9, 11, 12 * --- | 1,9 | C 07 D 249/12<br>A 01 N 43/653//<br>C 07 C 133/02<br>C 07 C 159/00 |
| A | EP-A-0 157 259 (CIAB-GEIGY A.G.) * examples 2, 6, 8, 14 * --- | 1,9 | |
| A | DE-A-1 943 915 (ROHM AND HAAS CO.) * claims 1, 19, 29 * --- | 1,5,7 | |
| A | GB-A-2 056 971 (NIHON NOHYAKU CO., LTD.) ----- | | |

TECHNICAL FIELDS SEARCHED (Int. Cl.4)

C 07 D 249/00
A 01 N 43/00

The present search report has been drawn up for all claims

| Place of search | Date of completion of the search | Examiner |
|---|---|---|
| BERLIN | 09-02-1987 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document